# EUROPEAN PATENT SPECIFICATION

(11) **EP 0 374 885 B1**
(45) Date of publication and mention of the grant of the patent: **04.05.1994**
(21) Application number: 89123558.2
(22) Date of filing: 20.12.1989
(51) Int. Cl.: C07D 307/46, C07D 307/58, C07D 307/64, A61K 31/34

(54) **Antiretroviral aryloxy substituted furan ketones**
Antiretrovirale aryloxy-substituierte Furanketone
Cétones furanniques aryloxy substituées antirétrovirales

(30) Priority: 21.12.1988 US 287817
(43) Date of publication of application: 27.06.1990
(73) Proprietor: MERRELL DOW PHARMACEUTICALS INC., Cincinnati Ohio 45215-6300 (US)
(72) Inventor: Parker, Roger A., Cincinnati Ohio 45231 (US); Sunkara, Sai P., Cincinnati Ohio 45236 (US)
(74) Representative: Sgarbi, Renato

(56) References cited:
- EP-A- 0 082 005
- EP-A- 0 150 118
- US-A- 4 011 334
- US-A- 4 644 009
- PATENT ABSTRACTS OF JAPAN vol. 9, no. 91 (C-277) (1814), 19 April 1985; & JP-A- 59 225135 (SUMITOMO KAGALU KOGYO) 18-12-1984

## Description

### FIELD OF INVENTION

The present invention relates to the use of certain aryloxy substituted furan alkyl ketones in the treatment of retroviral infections including HIV infections.

### BACKGROUND OF THE INVENTION

A great deal of research is currently underway to develop treatments and cures for viral infections in humans and in animals. Notably the incidence of acquired immune deficiency syndrome (AIDS) and AIDS related complex (ARC) in humans is increasing at an alarming rate. The five year survival rate for those with AIDS is dispiriting and AIDS patients, whose immune systems have been seriously impaired by the infection, suffer from numerous opportunistic infections including Kaposi's sarcoma and *Pneumocystis carninii* pneumonia. No cure is known and current treatments are largely without adequate proof of efficacy and have numerous untoward side effects. Fear of the disease has resulted in social ostracism of and discrimination against those having or suspected of having the disease.

Retroviruses are a class of ribonucleic acid (RNA) viruses that replicate by using reverse transcriptase to form a strand of complementary DNA (cDNA) from which a double stranded, proviral DNA is produced. This proviral DNA is then randomly incorporated into the chromosomal DNA of the host cell. Further transcription and translation of the integrated viral genome DNA results in viral replication through the synthesis of virus specific RNA and proteins.

Many of the known retroviruses are oncogenic or tumor causing. Indeed the first two human retroviruses discovered, denoted human T-cell leukemia virus I and II or HTLV-I and II, were found to cause rare leukemias in humans after infection of T-lymphocytes. The third such human virus to be discovered, HTLV-III, now referred to as HIV, was found to cause cell death after infection of T-lymphocytes and has been identified as the causative agent of acquired immune deficiency syndrome (AIDS) and AIDS related complex (ARC).

Among the substances previously shown to have activity against HIV and other retroviruses are such diverse compounds as azidothymidine, castanospermine, and heparin.

The applicants have now discovered that certain substituted furan ketones, more specifically furan ketones substituted at the 5-position of the furan ring by phenoxyalkyl and naphthalenyloxyalkyl moieties bonded to the furan ring either directly, through an ether or thioether bridge, or through an oxymethyl or thiomethyl bridge, are useful in the treatment of various retroviral infections including in the treatment of AIDS and ARC resulting from infection by HIV or other retroviruses.

### SUMMARY OF THE INVENTION

The anti-retrovirus compounds of this invention have the general Formula I 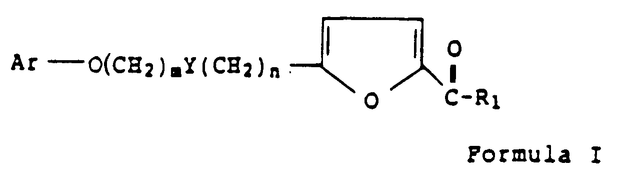
In the above general Formula I, Y is a bond, oxygen or divalent sulfur, n is 0 or 1, Ar is phenyl or naphthalenyl, m is from 4 to 10, and R₁ is C₁₋₆ alkyl.

### DETAILED DESCRIPTON OF THE INVENTION

In the above general Formula I, when Ar is naphthalenyl, the compounds have the general Formula II 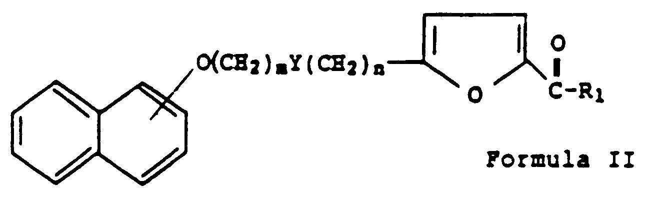
wherein the naphthalenyl substituent may be attached to the oxygen atom through the 1- or 2-position.

In the above general Formula I, when Ar is phenyl, the compounds have the general formula III. 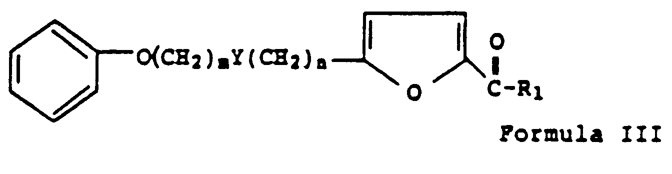
The linear, saturated carbon chain linking the ether with Y may range in length from 4 to 8 carbon atoms. Compounds having a chain length of 6 to 8 methylene units are preferred, with a chain length of 6 methylene units being most preferred.

Illustrative examples of straight or branched lower alkyl groups of from 1 to 6 carbon atoms which R₁ may represent are methyl, ethyl, n-propyl, isopropyl, n-butyl, tert-butyl, neopentyl, and n-hexyl.

The naphthalenyloxy substituted compounds of general Formula II represent a preferred embodiment of this invention. Of the compounds of general Formula I, those wherein Y is oxygen are more preferred. Also, the compounds of general Formula I wherein R₁ is a straight chain alkyl are preferred over the branched chain alkyl derivatives. Compounds wherein R₁ is methyl are particularly preferred. Also, the compounds of general Formula I wherein m is from 6-8 and wherein m is from 5-7 are preferred, 6 being most preferred. Another preferred embodiment of this invention is a pharmaceutical composition for the treatment of retrovirus infection comprising a compound of Formula I and a pharmaceutically acceptable carrier.

Another preferred embodiment of this invention is the use of compounds of general Formula I as antiretrovirus agents. The use of compounds of general Formula I wherein R₁ is a straight chain alkyl group are preferred, with R₁ as methyl being more preferred. Another preferred embodiment is the use of compounds of general Formula I as antiretrovirus agents wherein Ar is naphthalenyl. The use of compounds of general Formula I wherein Y is oxygen or sulfur is another preferred embodiment, with Y as oxygen being more preferred.

Illustrative examples of compounds of general Formula I are the following:
methyl 5-[6-(2-naphthalenyloxy)hexyloxy]-2-furyl ketone,
ethyl 5-[8-(1-naphthalenyloxy)octyloxy]-2-furyl ketone,
n-propyl 5-[7-(1-naphthalenyloxy)heptylthio]-2-furyl ketone,
methyl 5-[(6-(2-naphthalenyloxy)hexyl)oxymethyl]-2-furyl ketone,
isopropyl 5-[(4-(2-naphthalenyloxy)butyl)thiomethyl]-2-furyl ketone,
methyl [5-(2-naphthalenyloxy)pentyl]-2-furyl ketone,
methyl 5-(6-phenoxyhexyloxy)-2-furyl ketone,
ethyl 5-(10-phenoxydecyloxy)-2-furyl ketone,
n-propyl 5-(8-phenoxyoctylthio)-2-furyl ketone,
methyl 5-(9-phenoxynonlyoxymethyl)-2-furyl ketone,
isopropyl 5-(7-phenoxyheptylthiomethyl)-2-furyl ketone,
methyl 5-(6-phenoxyhexyl)-2-furyl ketone,
The ability of the furan ketone derivatives of this invention to act as anti-retroviral agents can be demonstrated by their ability to inhibit the growth and replication of murine leukemia virus, an oncogenic retrovirus, as determined by an *in vitro* XC plaque assay. This assay was performed according to the method of Rowe *et al.* (Virology, 1970, 42, 1136-39) as previously described by L. Hsu, *et al*. (J. Virological Methods, 1980, 1, 167-77) and T. L. Bowlin and M. R. Proffitt (J. Interferon Res., 1983, 3(1), 19-31). Mouse SC-1 cells (fibroblast) (10⁵) were seeded into each well of 6-well cluster plates (Costar #3506) in 4 ml Minimum Essential Medium (MEM) with 10% Fetal Calf Serum (FCS). Following an 18 hour incubation period (37°C), Moloney murine leukemia virus (MoLV) was applied at a predetermined titer to give optimal (i.e. countable) numbers of virus plaques. Methyl 5-[6-(2-naphthalenyloxy)hexyloxy]-2-furyl ketone was added 2 hours prior to addition of the virus at various concentrations. Three days later the culture medium was removed, the SC-1 cell monolayers were exposed to UV irradiation (1800 ergs), and rat XC cells (10⁶) were seeded into each well in 4 ml MEM. Following an additional 3 day incubation (37°C), these cells were fixed with ethyl alcohol (95%) and stained with 0.3% crystal violet. Plaques were then counted under low magnification. The IC₅₀, i,e, the concentration giving a 50% inhibition of virus plaque growth, was below 1 µg/ml, indicating the exceptional antiviral activity of the tested compound of this invention.

The furan ketone derivatives of this invention can be used to treat a number of diseases and conditions known to be caused by retroviruses including those diseases and conditions caused by murine leukemia virus, feline leukemia virus, avian sarcoma virus, human immuno-deficiency virus (HIV), HTLV-I, and HTLV-II. Those experienced in this field are readily aware of the circumstances requiring anti-retroviral therapy. Applicants consider the use of the furan ketone derivatives of this invention to treat HIV infections in humans to be of most importance. The term "patient" used herein is taken to mean mammals such as primates, including humans, sheep, horses, cattle, pigs, dogs, cats, rats and mice, and birds.

The amount of the furan ketone derivative of formula I to be administered can vary widely according to the particular dosage unit employed, the period of treatment, the age and sex of the patient treated, the nature and extent of the disorder treated, and the particular furan ketone derivative selected. Moreover the furan ketone derivative can be used in conjunction with other agents known to be useful in the treatment of retroviral diseases and agents known to be useful to treat the symptoms of and complications associated with diseases and conditions caused by retroviruses. The anti-retrovirally effective amount of furan ketone derivative of formula I to be administered will generally range from 15 mg/kg to 500 mg/kg. A unit dosage may contain from 25 to 500 mg of the furan ketone derivative, and can be taken one or more times per day. The furan ketone derivative can be administered with a pharmaceutical carrier using conventional dosage unit forms either orally or parenterally.

The preferred route of administration is oral administration. For oral administration the furan ketone derivative can be formulated into solid or liquid preparations such as capsules, pills, tablets, troches, lozenges, melts, powders, solutions, suspensions or emulsions. The solid unit dosage forms can be capsules, which can be of the ordinary hard- or soft-shelled gelatin type containing, for example, surfactants, lubricants, and inert fillers such as lactose, sucrose, calcium phosphate and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration, such as potato starch, alginic acid, corn starch and guar gum, lubricants intended to improve the flow of tablet granulations and to prevent the adhesion of tablet material to the surfaces of the tablet dies and punches, for example, talc, stearic acid, or magnesium, calcium or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptable surfactant, suspending agent, or emulsifying agent.

The furan ketone derivatives of this invention may also be administered parenterally, that is, subcutaneously, intravenously, intramuscularly or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol or hexadecyl alcohol, glycols such as propylene glycol or polyethylene glycol, glycerol ketals such as 2,2-dimethyl-1,3-dioxolane-4-methanol, ethers such as poly(ethylene glycol) 400, an oil, a fatty acid, a fatty acid ester or glyceride, or an acetylated fatty acid glyceride, with or without the addition of a pharmaceutically acceptable surfactant such as a soap or a detergent, a suspending agent such as pectin, carbomers, methylcellulose, hydroxypropylmethylcellulose or carboxymethylcellulose, or an emulsifying agent, and other pharmaceutical adjuvants. Illustrative of oils which can be used in the parenteral formulations of this invention are those of petroleum, animal, vegetable, and synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl β-aminopropionates and 2-alkylimidazoline quarternary ammonium salts, as well as mixtures. The parenteral compositions of this invention will typically contain from 0.5 to 25% by weight of the furan ketone derivative of formula 1 in solution. Preservatives and buffers may also be used advantageously. In order to minimize or eliminate irritation at the site of injection, such compositions may contain a non-ionic surfactant having a hydrophile-lipophile balance (HLB) of from 12 to 17. The quantity of surfactant in such formulations ranges from 5 to 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The ketone compounds of general Formula I may be prepared by treating one equivalent of the corresponding carboxylic acid derivatives with two equivalents of alkyllithium, wherein the alkyl group corresponds to the desired R₁ substituent, as generally described by Fieser and Fieser, Reagents for Organic Synthesis, J. Wiley and Sons, Inc., New York, p. 688 (1967). This reaction is suitably carried out in solvents such as ether, tetrahydrofuran, p-dioxane, dimethoxyethane or diethyleneglycol dimethylether at temperatures of from -10°C to the reflux temperature of the solvent for from ½ hour to 10 hours.

The ketone compounds of general Formula I may also be prepared by the reaction of alkyl magnesium bromide wherein the alkyl group corresponds to the desired R₁ substituent and the imidazolide derivative of an appropriately 5-ArO(CH₂)ₘ Y(CH₂)ₙ substituted 2-furancarboxylic acid derivative wherein Ar, Y, m and n have the meanings defined in general Formula I. This reaction is carried out in a solvent such as ether, tetrahydrofuran, dioxane, dimethoxyethane, or acetonitrile. The reaction mixture is initially cooled to -10°C, after which the temperature is elevated to from about 25°C to the reflux temperature of the solvent, and the reaction time varies from ½ hour to 10 hours. The imidazolide derivative is obtained by treating an appropriate 5-ArO(CH₂)ₘY(CH₂)ₙ substituted 2-furancarboxylic acid derivative with N,N′-carbonyldiimidazole or by treatment of the 5-Ar(CH₂)ₘY(CH₂)ₙ substituted 2-furancarboxylic acid chloride, obtained by treating the substituted carboxylic acid with thionyl chloride, with two equivalents of imidazole, as generally described by H.A. Staab, Angew. Chem. Internat. Edit. 1, 351 (1962).

The compounds of general Formula I may also be prepared by a Friedel-Crafts acylation of an appropriately ArO(CH₂)ₘ(CH₂)ₙ substituted furan, wherein Ar, Y, m and n have the meanings defined in general Formula I, with an acyl halide of the formula 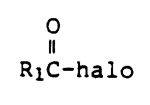
wherein halo is halogen, preferably chlorine or bromine and R₁ has the meaning defined above. This reaction is carried out in the presence of an acid catalyst, for example, borontrifluoride-etherate, stannic chloride, zinc chloride, hydrocloric acid or orthophosphoric acid, and optionally in the presence of a solvent, for example, methylene chloride, nitromethane or benzene. Suitable temperatures for this reaction may vary from -20°C to the reflux temperature of the solvent and the reaction time varies from ½ hour to 10 hours.

The ArO(CH₂)ₘO- and ArO(CH₂)ₘS- substituted furancarboxylic acid derivative used herein can be prepared by aromatic nucleophilic substitution as generally described in J. March, Advanced Organic Chemistry: Reactions, Mechanisms and Structure, McGraw-Hill, p. 500 (1968), as outlined below 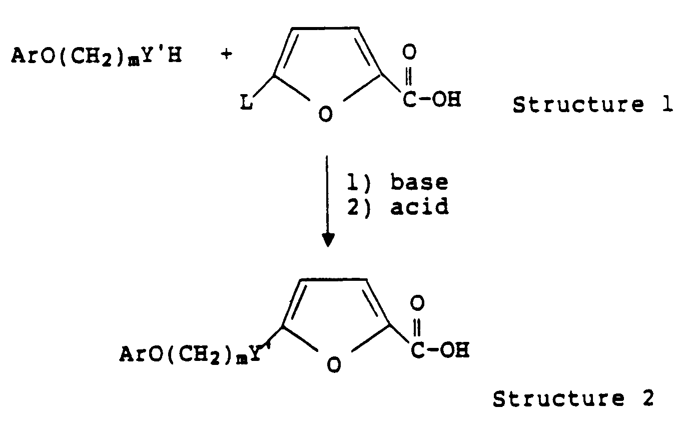
In the above general reaction, Ar and m have the meanings defined in general Formula I, Y′ represents oxygen or divalent sulfur, and L represents a leaving group, such as nitro, fluoro, chloro, bromo or iodo, the preferred leaving group being chloro.

The above reaction may be carried out with or without a solvent. Suitable solvents for the reaction include benzene, xylene, toluene, chlorinated hydrocarbon solvents such as chlorobenzene, ethers such as bis(2-methoxyethyl)ether, 1,2-dimethoxyethane or anisole, hexamethylphosphoric triamide (HMPA), dimethylformamide, dimethylacetamide, 1-methyl-2-pyrrolidone, or pyridine. Preferred solvents are xylene, toluene and dimethylacetamide. Copper metal or a salt such as cuprous chloride may optionally be added to the reaction. Suitable bases for the reaction include sodium or potassium metal, sodium hydride, potassium amide, potassium tert-butoxide or other strong bases such as potassium carbonate, potassium hydroxide, sodium hydroxide and sodium carbonate. The temperature of the reaction varies from about 25°C to the reflux temperature of the solvent, and the reaction time varies from 1 hour to 7 days. Following completion of the reaction, the carboxylate salt derivative is treated with a mineral or organic acid to give compounds of structure 2.

The furoic acid derivatives represented by compounds of structure 1 may be prepared by several methods, as described in The Furans, by A.P. Dunlop and F.N. Peters, Reinhold Publishing Corp., pp. 80-169 (1953).

The 5-ArO(CH₂)ₘY(CH₂)ₙ substituted furan carboxylic acid derivatives employed herein wherein Y is a bond and n is 0 can be prepared by treating a compound of the structure 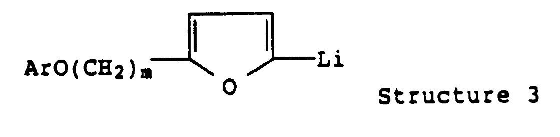
wherein Ar and m have the meanings defined in general Formula I with dry ice followed by the addition of water by procedures known in the art. The compounds of structure 3 are obtained by metalation of the appropriately substituted furan with butyllithium.

ArO(CH₂)ₘY- substituted furan derivatives wherein Y is a bond can be obtained by the reaction of 2-lithiofuran, prepared by treating furan with butyllithium, with an ArO(CH₂)ₘ halide wherein Ar and m have the meanings defined in general Formula I by procedures generally known in the art. The ArO(CH₂)ₘ halides used herein are commercially available or may be prepared by well-known procedures.

Likewise, the ArO(CH₂)ₘ-Y′CH₂- substituted furan carboxylic acid derivatives used herein can be prepared by metalation followed by addition of carbon dioxide (carboxylation) as illustrated below. 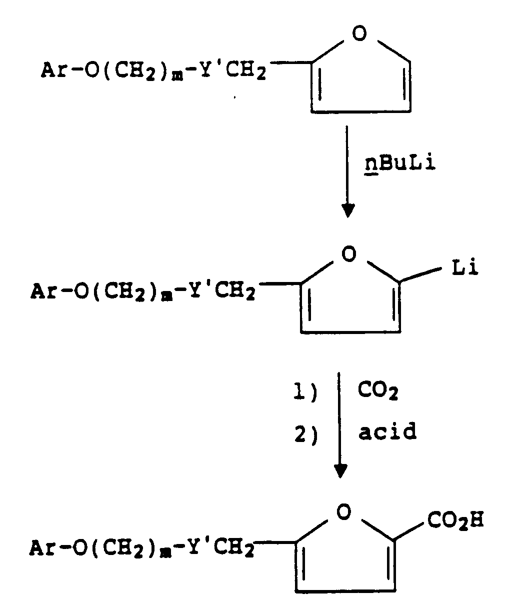
The ArO(CH₂)ₘOCH₂- and ArO(CH₂)mSCH₂- substituted furans can be obtained by reaction of furfuryl alcohol or furfuryl mercaptan by Williamson ether synthesis (J. March, "Advanced Organic Chemistry - Reactions, Mechanisms and Structure," McGraw-Hill Book Company, New York, 1968, p. 316). The reaction is illustrated in the following reaction scheme: 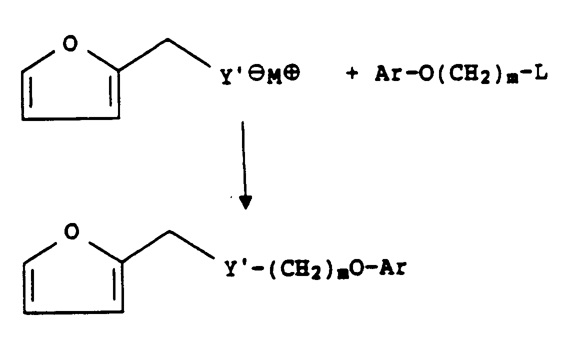
In the above reaction sequence, L represents a halogen atom, such as chlorine, bromine or iodine, or a sulfonate ester, such as methanesulfonate or p-toluenesulfonate; M⁺ represents a metal salt such as lithium, sodium, potassium, silver or mercury, and Ar, m and Y′ have the meanings described above.

A furfuryl alkoxide salt, conveniently formed in situ by addition of a base such as sodium methoxide, potassium carbonate, sodium hydride or potassium hydroxide to the corresponding alcohol or mercaptan, is reacted with the desired aryl alkyl ether bearing a leaving group on the terminal carbon atom. The leaving group is displaced, resulting in the formation of a carbon-oxygen or carbon-sulfur ether bond.

The L-substituted aryl alkyl ethers used in the sequence are generally available commercially or by well-known, conventional synthetic methods.

The ArO(CH₂)ₘY′CH₂- substituted furan carboxylic acid derivative used herein may also be prepared from an ester of 5-methylfuran carboxylic acid by a Williamson ether synthesis as shown in the reaction scheme below: 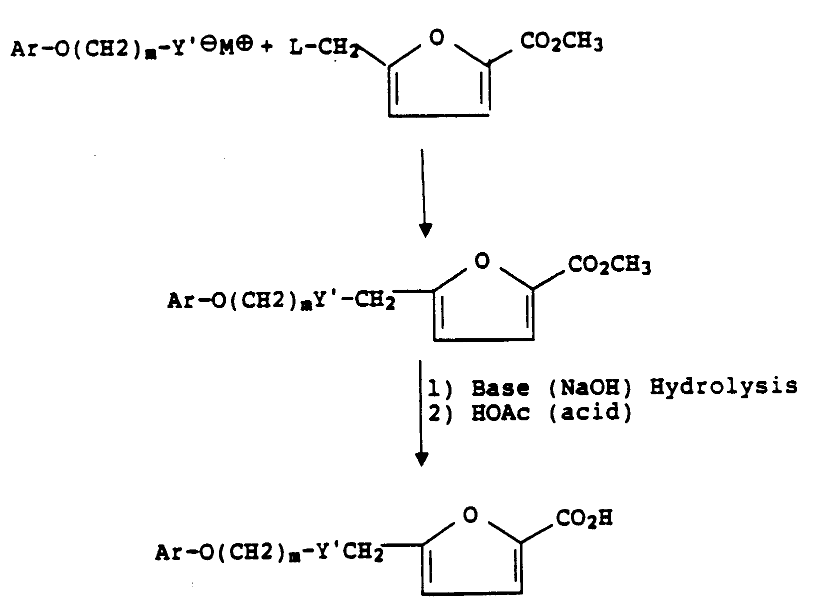
An alkoxide salt, conveniently formed in situ by addition of a base such as sodium methoxide, potassium carbonate, sodium hydride or potassium hydroxide to the aryloxyalkyl alcohol or mercaptan having the desired ArO(CH₂)ₘ skeleton, is reacted with a 5-methylfuroic acid ester bearing a leaving group on the methyl carbon atom. The leaving group is displaced, resulting in the formation of a carbon-oxygen or carbon-sulfur ether bond, and the resulting 5-ArO(CH₂)ₘY′(CH₂)ₙ-substituted 2-furoic acid ester is hydrolyzed to the desired acid by methods well known in the art.

The substituted furoic acid esters used in the sequence are generally available commercially or by well-known, conventional synthetic methods. The aryloxyalkyl alcohols and mercaptans may be prepared by well-known, conventional synthetic methods, for example by the Williamson reaction between a phenoxide or naphthoxide salt and an alkanol substituted by a leaving group on the terminal carbon atom, as illustrated in the following reaction scheme:

ArO^{⊖}M^{⊕} + L-(CH₂)ₘY′H→ ArO(CH₂)ₘY′H

In the above reaction sequence, L represents a halogen atom, such as chlorine, bromine or iodine, or a sulfonate ester such as methanesulfonate or p-toluenesulfonate; M⁺ represents a metal ion such as lithium, sodium, potassium, silver or mercury; and Ar and n are as defined for Formula I. The starting naphthols and phenol which are the precursors of the naphthoxide and phenoxide salts are commercially available. The ω-substituted linear alcohols and mercaptans, III, used in the sequence are also generally available commercially or by well-known, conventional synthetic methods. For example, the α,ω-diol may be converted to the ω-haloalcohol using the triphenylphosphine and carbon tetrahalide.

The Williamson reaction may be carried out with or without solvents. Suitable solvents for the reaction incude lower alcohols, such as ethanol and isopropanol, ketones such as acetone and butanone, or amides such as dimethylformamide and dimethylacetamide. Other suitable solvents include dimethylsulfoxide, acetonitrile, dimethoxyethane, tetrahydrofuran and toluene.

The temperature of the reaction may vary from 0°C to the reflux temperature of the solvent, and the reaction time may vary from 0.5 hour to 80 hours.

The reaction is conveniently worked up by extraction of the product into an organic solvent such as ether, dichloromethane, chloroform or toluene, washing with brine, drying over sodium or magnesium sulfate, and evaporation of the solvent. Purification is generally effected by distillation or crystallization from a suitable solvent.

### EXAMPLE 1

### Methyl 5-[6-(2-naphthalenyloxy)hexyloxy]-2-furyl ketone

A mixture of 50.0 g (0.348 mole) of 2-naphthol, 18.8 g (0.348 mole) of sodium methoxide, 2.0 g of sodium iodide and 800 ml of dimethylacetamide was stirred at room temperature for 1 hour, 47.5 g (0.348 mole) of 6-chlorohexanol was added. The mixture was heated to reflux with stirring for two hours, allowed to cool and poured into 3 liters of water and extracted with diethylether. The ether layer was evaporated to dryness to give a solid residue which was recrystallized from methanol to give 13.3 g of 6-(2-naphthalenyloxy)hexanol, mp = 64-65°C.

A mixture of 12.2 g (0.05 mole) of 6-(2-naphthalenyloxy)hexanol, 4.8 g (0.10 mole) of 50% sodium hydride in oil and 200 ml of toluene was stirred at room temperature for 1 hour, 50 ml of hexamethylphosphoric triamide (HMPA) was added and the mixture refluxed for 2 and 1/2 hours. 7.3 g (0.05 mole) of 5-chloro-2-furancarboxylic acid was added and the mixture was refluxed for sixteen hours, then allowed to cool and was diluted with water. The mixture was acidifed by addition of glacial acetic acid and extracted with diethylether. The ether layer was washed with water and filtered. Evaporation of the mixture to about 100 ml volume gave 8.6 g (43%) tan solid 5-[6-(2-napthalenlyoxy)hexyloxy)]-2-furancarboxylic acid, mp 127-129°C.

A mixture of 5.0 g (0.0125 mole) of 5-[6-(2-napthaleniloxy hexyloxy]-2-furancarboxylic acid and 100 ml of anhydrous ether was stirred at room temperature. Methyllithium (18 ml of a 1.55 molar solution, 0.028 mole) was added dropwise with stirring over 20 minutes. The mixture stood for 30 minutes, then 30 ml of tetrahydrofuran was added and the mixture refluxed. 10 ml of hexamethylphosphoric triamide (HMPA) was added and the reaction stirred at room temperature for 2 hours. Saturated ammonium chloride in water (200 ml) was added and the layers were separated. The ether layer was washed with water and filtered through alumina and was evaporated to dryness under reduced pressure to give 4.6 g of a light brown solid. Recrystallization from acetonitrile and then from ethanol gave 1.1 g of a light tan solid, methyl 5-[6-(2-napthalenyloxy)hexyloxy]-2-furyl ketone, mp = 94-100°C.

### EXAMPLE 2

### Methyl 5-(6-phenoxyhexyloxy)-2-furyl ketone

When phenol was substituted for 2-naphthol in the procedure of Example 1, methyl 5-(6-phenoxyhexyloxy)-2-furyl ketone was obtained, mp = 80-82°C.

### EXAMPLE 3

### Methyl 5-(10-phenoxydecyloxy)-2-furyl ketone

When phenol was substituted for 2-naphthol and 1-chlorodecanol was substituted for 6-chlorohexanol in the procedure of Example 1, methyl 5-(10-phenoxydecyloxy)-2-furyl ketone was obtained, mp = 74-77°C.

### EXAMPLE 4

### Solution

| | |
|---|---|
| Methyl 5-[6-(2-naphthalenyloxy)hexyloxy]-2-furyl ketone | 0.85 g |
| Alcohol | 78.9 ml |
| Isopropyl Myristate | 5.0 g |
| Polyethylene Glycol 400 (Av. M.W. 400) | 10.0 g |
| Purified Water sufficient to make | 100 ml |

Combine the alcohol, isopropyl myristate and polyethylene glycol 400 and dissolve the drug substance therein. Add sufficient purified water to give 100 ml.

### EXAMPLE 5

### Tablet

| | For 15,000 |
|---|---|
| Methyl 5-[6-(2-naphthalenyloxy)hexyloxy]-2-furyl ketone | 75 g |
| Lactose | 1.216 kg |
| Corn Starch | 0.3 kg |
| Mix the active ingredient, the lactose and corn starch uniformly. Granulate with 10% starch paste. Dry to a moisture content of about 2.5%. Screen through a No. 12 mesh screen. Add and mix the following: | |
| Magnesium | 0.015 kg |
| Corn Starch sufficient to make | 1.725 kg |

Compress on a suitable tablet mechine to a weight of 0.115 g/tablet.

### EXAMPLE 6

### Soft Gelatin Capsule

| | |
|---|---|
| Methyl 5-(6-phenoxyhexyloxy)-2-furyl ketone | 0.25 kg |
| Polysorbate 80 (Polyoxyethylene (20) sorbitan mono-oleate) | 0.25 kg |
| Corn Oil sufficient to make | 25.0 kg |

Mix and fill into 50,000 soft gelatin capsules.

## Claims (Claims for the following Contracting State(s): AT, BE, FR, CH, DE, GB, IT, LU, NL, SE, LI)

1. A compound of the formula 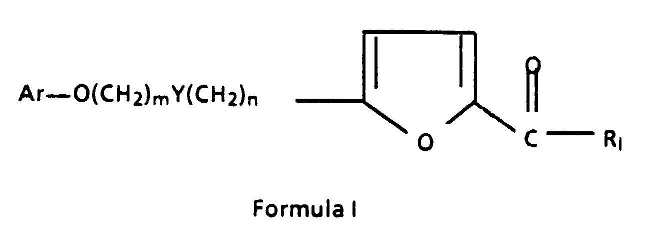 wherein Y represents a bond, oxygen or divalent sulfur; Ar represents phenyl or naphthalenyl; n is 0 or 1; m is an integer of from 4 to 10; and R₁ represents C₁₋₆ alkyl.

2. A compound according to Claim 1 wherein Ar is 1- or 2- naphthalenyl.

3. A compound according to Claim 1 wherein Y is oxygen.

4. A compound according to Claim 1 wherein m is an integer of from 5 to 7.

5. A compound according to Claim 1 wherein m is 6.

6. A compound according to Claim 1 wherein the compound is methyl 5-[6-(2-naphthalenyloxy)hexyloxy]-2-furyl ketone.

7. A compound according to Claim 1 wherein the compound is methyl 5-(6-phenoxyhexyloxy)-2-furyl ketone.

8. A compound according to Claim 1 wherein the compound is methyl 5-(10-phenoxydecyloxy)-2-furyl ketone.

9. A compound as in Claim 1 for use as a medicament.

10. A compound for use as a medicament according to Claim 9 wherein Ar is 1- or 2- naphthalenyl.

11. A compound for use as a medicament according to Claim 9 wherein Y is oxygen.

12. A compound for use as a medicament according to Claim 9 wherein m is an integer of from 5 to 7.

13. A compound for use as a medicament according to Claim 9 wherein m is 6.

14. A compound for use as a medicament according to Claim 9 wherein the compound is methyl 5-[6-(2-naphthalenyloxy)hexyloxy]-2-furyl ketone.

15. A compound for use as a medicament according to Claim 9 wherein the compound is methyl 5-(6-phenoxyhexyloxy)-2-furyl ketone.

16. A compound for use as a medicament according to Claim 9 wherein the compound is methyl 5-(10-phenoxydecyloxy)-2-furyl ketone.

17. Use of a compound according to Claim 1 for the manufacture of a medicament for treating a retroviral infection in a patient in need thereof.

18. Use of a compound according to Claim 17 selected from:
a compound according to Claim 1 wherein Ar is 1- or 2-naphthalenyl;
a compound according to Claim 1 wherein Y is oxygen;
a compound according to Claim 1 wherein m is an integer of from 5 to 7;
a compound according to Claim 1 wherein m is 6;
the compound according to Claim 1 that is methyl 5-[6-(2-naphthalenyloxy)hexyloxy]-2-furyl ketone;
the compound according to Claim 1 that is methyl 5-(6-phenoxyhexyloxy)-2-furyl ketone;
the compound according to Claim 1 that is methyl 5-(10-phenoxydecyloxy)-2-furyl ketone.

19. Use of a compound according to any of claims 1 to 11 wherein the retroviral infections include those diseases caused by murine leukemia virus, feline leukemia virus, avian sarcoma virus, human immunodeficiency virus (HIV), HTLV-I, and HTLV-II.

20. A pharmaceutical composition comprising the compound of claim 1 in admixture with a pharmaceutically acceptable carrier.

21. A pharmaceutical composition according to claim 20 comprising a compound selected from:
a compound according to Claim 1 wherein Ar is 1- or 2- naphthalenyl;
a compound according to Claim 1 wherein Y is oxygen;
a compound according to Claim 1 wherein m is an integer of from 5 to 7;
a compound according to Claim 1 wherein m is 6;
the compound according to Claim 1 that is methyl 5-[6-(2-naphthalenyloxy)hexyloxy]-2-furyl ketone;
the compound according to Claim 1 that is methyl 5-(6-phenoxyhexyloxy)-2-furyl ketone;
the compound according to Claim 1 that is methyl 5-(10-phenoxydecyloxy)-2-furyl ketone.

## Claims (Claims for the following Contracting State(s): ES, GR)

1. A method for the preparation of a compound of the formula 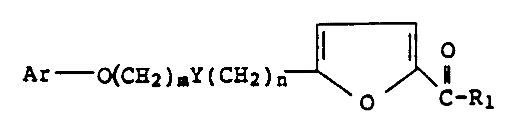 wherein Y represents a bond, oxygen or divalent sulfur; Ar represents phenyl or naphthalenyl; n is 0 or 1; m is an integer of from 4 to 10; and R₁ represents C₁₋₆ alkyl; which comprises:
a. treating one equivalent of a carboxylic acid derivative of the formula 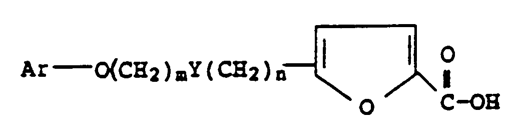 wherein Y, Ar, m and n have the meanings defined above, with two equivalents of alkyllithium, wherein the alkyl group corresponds to the desired R₁ substituent, in a solvent selected from ether, tetrahydrofuran, p-dioxane, dimethoxyethane and diethyleneglycol dimethylether, at a temperature of from -10°C to the reflux temperature of the solvent; or
b. combining an alkyl magnesium bromide of the formula R₁MgBr and the imidazolide derivative of a 2-furancarboxylic acid of the formula 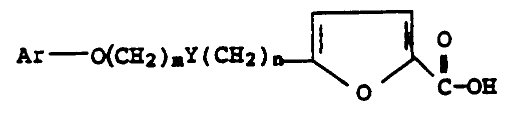 wherein Y, Ar, m and n have the meanings defined above, in a solvent selected from ether, tetrahydrofuran, dioxane, dimethoxyethane and acetonitrile, and heating the mixture to a temperature from 25°C to the reflux temperature of the solvent; or
c. reacting a ArO(CH₂)ₘY(CH₂)ₙ- substituted furan of the formula 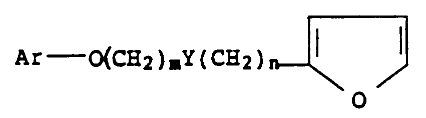 wherein Ar,m,n and Y have the meanings defined above, with an acyl halide of the formula 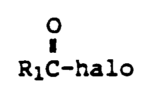 wherein halo is chlorine or bromine and R₁ has the meaning defined above, in the presence of an acid catalyst selected from boron trifluoride-etherate, stannic chloride, zinc chloride, hydrocloric acid and orthophosphoric acid.

2. A process according to Claim 1 wherein Ar is 1- or 2-naphthalenyl.

3. A process according to Claim 1 wherein Y is oxygen.

4. A process according to Claim 1 wherein m is an integer of from 5 to 7.

5. A process according to Claim 1 wherein m is 6.

6. The process according to Claim 1 wherein the compound is methyl 5-[6-(2-naphthalenyloxy)hexyloxy]-2-furyl ketone.

7. The process according to Claim 1 wherein the compound is methyl 5-(6-phenoxyhexyloxy)-2-furyl ketone.

8. The process according to Claim 1 wherein the compound is methyl 5-(10-phenoxydecyloxy)-2-furyl ketone.

## Patentansprüche (Patentansprüche für folgende(n) Vertragsstaat(en): AT, BE, FR, CH, DE, GB, IT, LU, NL, SE, LI)

1. Verbindung der Formel 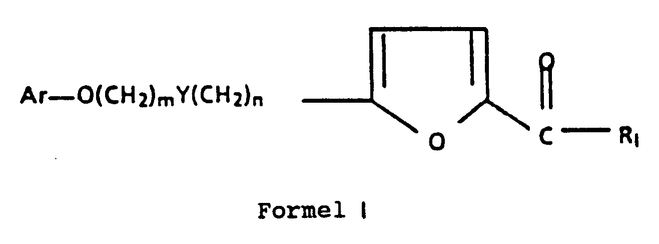 in der Y eine Bindung, ein Sauerstoffatom oder ein zweiwertiges Schwefelatom bedeutet; Ar eine Phenyl- oder Naphthalinylgruppe bedeutet; n die Zahl 0 oder 1 ist; m eine ganze Zahl von 4 bis 10 ist; und R₁ einen C₁₋₆-Alkylrest bedeutet.

2. Verbindung nach Anspruch 1, worin Ar eine 1- oder 2-Naphthalinylgruppe bedeutet.

3. Verbindung nach Anspruch 1, worin Y ein Sauerstoffatom ist.

4. Verbindung nach Anspruch 1, worin m eine ganze Zahl von 5 bis 7 ist.

5. Verbindung nach Anspruch 1, worin m 6 ist.

6. Verbindung nach Anspruch 1, wobei die Verbindung Methyl-5-[6-(2-naphthalinyloxy)hexyloxy]-2-furylketon ist.

7. Verbindung nach Anspruch 1, wobei die Verbindung Methyl-5-(6-phenoxyhexyloxy)-2-furylketon ist.

8. Verbindung nach Anspruch 1, wobei die Verbindung Methyl-5-(10-phenoxydecyloxy)-2-furylketon ist.

9. Verbindung nach Anspruch 1 zur Verwendung als Medikament.

10. Verbindung zur Verwendung als Medikament nach Anspruch 9, worin Ar eine 1- oder 2-Naphthalinylgruppe bedeutet.

11. Verbindung zur Verwendung als Medikament nach Anspruch 9, worin Y ein Sauerstoffatom bedeutet.

12. Verbindung zur Verwendung als Medikament nach Anspruch g, worin m eine ganze Zahl von 5 bis 7 ist.

13. Verbindung zur Verwendung als Medikament nach Anspruch 9, worin m 6 ist.

14. Verbindung zur Verwendung als Medikament nach Anspruch 9, worin die Verbindung Methyl-5-[6-(2-naphthalinyloxy)hexyloxy]-2-furylketon ist.

15. Verbindung zur Verwendung als Medikament nach Anspruch 9, worin die Verbindung Methyl-5-(6-phenoxyhexyloxy)-2-furylketon ist.

16. Verbindung zur Verwendung als Medikament nach Anspruch 9, worin die Verbindung Methyl-5-(10-phenoxydecyloxy)-2-furylketon ist.

17. Verwendung einer Verbindung nach Anspruch 1 zur Herstellung eines Medikaments zur Behandlung einer retroviralen Infektion bei einem Patienten, der es benötigt.

18. Verwendung einer Verbindung nach Anspruch 17, nämlich:
eine Verbindung nach Anspruch 1, worin Ar eine 1- oder 2-Naphthalinylgruppe bedeutet;
eine Verbindung nach Anspruch 1, worin Y ein Sauerstoffatom bedeutet;
eine Verbindung nach Anspruch 1, worin m eine ganze Zahl von 5 bis 7 ist;
eine Verbindung nach Anspruch 1, worin m 6 ist.
die Verbindung nach Anspruch 1, welche Methyl-5-[6-(2-naphthalinyloxy)-hexyloxy]-2-furylketon ist.
die Verbindung nach Anspruch 1, welche Methyl-5-(6-phenoxyhexyloxy)-2-furylketon ist.
die Verbindung nach Anspruch 1, welche Methyl-5-(10-phenoxydecyloxy)]-2-furylketon ist.

19. Verwendung einer Verbindung nach einem der Ansprüche 1 bis 11, worin die retroviralen Infektionen solche Krankheiten einschließen, die durch Maus-Leukämie-Virus, Katzen-Leukämie-Virus, Vogel-Sarkom-Virus, menschliches Immunschwäche-Virus (HIV), HTLV-I und HTLV-II verursacht werden.

20. Arzneimittel, umfassend die Verbindung nach Anspruch 1 in einem Gemisch mit einem pharmazeutisch verträglichen Träger.

21. Arzneimittel nach Anspruch 20, umfassend eine Verbindung, nämlich:
eine Verbindung nach Anspruch 1, worin Ar eine 1- oder 2-Naphthalinylgruppe bedeutet;
eine Verbindung nach Anspruch 1, worin Y ein Sauerstoffatom bedeutet;
eine Verbindung nach Anspruch 1, worin m eine ganze Zahl von 5 bis 7 ist;
eine Verbindung nach Anspruch 1, worin m 6 ist.
die Verbindung nach Anspruch 1, welche Methyl-5-[6-(2-naphthalinyloxy)-hexyloxy]-2-furylketon ist.
die Verbindung nach Anspruch 1, welche Methyl-5-(6-phenoxyhexyloxy)-2-furylketon ist.
die Verbindung nach Anspruch 1, welche Methyl-5-(10-phenoxydecyloxy)]-2-furylketon ist.

## Patentansprüche (Patentansprüche für folgende(n) Vertragsstaat(en): ES, GR)

1. Verfahren zur Herstellung einer Verbindung der Formel 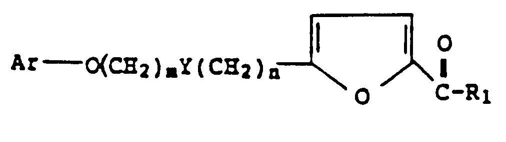 in der Y eine Bindung, ein Sauerstoffatom oder ein zweiwertiges Schwefelatom bedeutet; Ar eine Phenyl- oder Naphthalinylgruppe bedeutet; n 0 oder 1 ist; m eine ganze Zahl von 4 bis 10 ist; und R₁ einen C₁₋₆-Alkylrest bedeutet; umfassend:
a. Behandlung eines Äquivalents eines Carbonsäure-Derivats der Formel 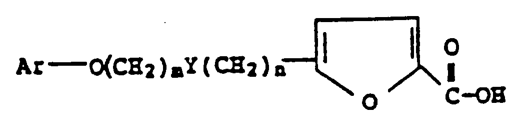 in der Y, Ar, m und n wie vorstehend definiert sind, mit zwei Äquivalenten Alkyllithium, wobei der Alkylrest dem gewünschten R₁-Substituenten entspricht, in einem Lösungsmittel, ausgewählt aus Ether, Tetrahydrofuran, p-Dioxan, Dimethoxyethan und Diethylenglycoldimethylether, bei einer Temperatur von -10°C bis zur Rückflußtemperatur des Lösungsmittels; oder
b. Zusammengeben eines Alkylmagnesiumbromids der Formel R₁MgBr und des Imidazolid-Derivats einer 2-Furancarbonsäure der Formel 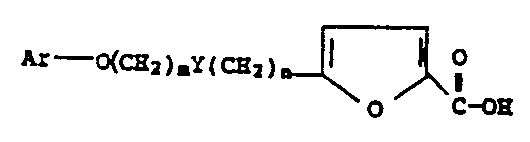 worin Y, Ar, m und n wie vorstehend definiert sind, in einem Lösungsmittel, ausgewählt aus Ether, Tetrahydrofuran, Dioxan, Dimethoxyethan und Acetonitril, und Erhitzen des Gemisches auf eine Temperatur von 25°C bis zur Rückflußtemperatur des Lösungsmittels; oder
c. Umsetzung eines ArO(CH₂)ₘ-Y(CH₂)ₙ-substituierten Furans der Formel 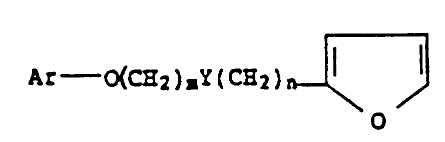 worin Ar, m, n und Y wie vorstehend definiert sind, mit einem Acylhalogenid der Formel 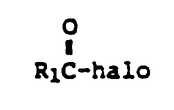 worin halo ein Chlor- oder Bromatom bedeutet und R₁ wie vorstehend definiert ist, in Gegenwart eines sauren Katalysators, ausgewählt aus Bortrifluorid-Etherat, Zinnchlorid, Zinkchlorid, Salzsäure und Orthophosphorsäure.

2. Verfahren nach Anspruch 1, worin Ar eine 1- oder 2-Naphthalinylgruppe bedeutet.

3. Verfahren nach Anspruch 1, worin Y ein Sauerstoffatom bedeutet;

4. Verfahren nach Anspruch 1, worin m eine ganze Zahl von 5 bis 7 ist;

5. Verfahren nach Anspruch 1, worin m 6 ist.

6. Verfahren nach Anspruch 1, worin die Verbindung Methyl-5-[6-(2-naphthalinyloxy)hexyloxy]-2-furylketon ist.

7. Verfahren nach Anspruch 1, worin die Verbindung Methyl-5-(6-phenoxyhexyloxy)-2-furylketon ist.

8. Verfahren nach Anspruch 1, worin die Verbindung Methyl-5-(10-phenoxydecyloxy)-2-furylketon ist.

## Revendications (Revendications pour l'(les) Etat(s) contractant(s) suivant(s): AT, BE, FR, CH, DE, GB, IT, LU, NL, SE, LI)

1. Composé de formule 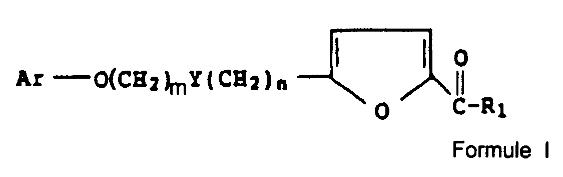 dans laquelle Y représente une liaison, un atome d'oxygène ou un atome de soufre divalent; Ar représente un groupe phényle ou naphtalényle; n vaut 0 ou 1; m est un nombre entier de 4 à 10; et R₁ représente un groupe alkyle en C₁ à C₆.

2. Composé selon la revendication 1, dans lequel Ar est un groupe 1- ou 2-naphtalényle.

3. Composé selon la revendication 1, dans lequel Y est l'atome d'oxygène.

4. Composé selon la revendication 1, dans lequel m est un nombre entier de 5 à 7.

5. Composé selon la revendication 1, dans lequel m vaut 6.

6. Composé selon la revendication 1, dans lequel le composé est la méthyl-5-[6-(2-naphtalényloxy)hexyloxy]-2-furylcétone.

7. Composé selon la revendication 1, dans lequel le composé est la méthyl-5-(6-phénoxyhexyloxy)-2-furylcétone,

8. Composé selon la revendication 1, dans lequel le composé est la méthyl-5-(10-phénoxydécyloxy)-2-furylcétone.

9. Composé selon la revendication 1 pour une utilisation en tant que médicament.

10. Composé pour une utilisation en tant que médicament selon la revendication 9, dans lequel Ar est un groupe 1- ou 2-naphtalényle.

11. Composé pour une utilisation en tant que médicament selon la revendication 9, dans lequel Y est l'atome d'oxygène.

12. Composé pour une utilisation en tant que médicament selon la revendication 9, dans lequel m est un nombre entier de 5 à 7.

13. Composé pour une utilisation en tant que médicament selon la revendication 9, dans lequel m vaut 6.

14. Composé pour une utilisation en tant que médicament selon la revendication 9, dans lequel le composé est la méthyl-5-[6-(2-naphtalényloxy)hexyloxy]-2-furylcétone.

15. Composé pour une utilisation en tant que médicament selon la revendication 9, dans lequel le composé est la méthyl-5-(6-phénoxyhexyloxy)-2-furylcétone.

16. Composé pour une utilisation en tant que médicament selon la revendication 9, dans lequel le composé est la méthyl-5-(10-phénoxydécyloxy)-2-furylcétone.

17. Utilisation d'un composé selon la revendication 1 pour la fabrication d'un médicament destiné à traiter une infection rétrovirale chez un patient en ayant besoin.

18. Utilisation d'un composé selon la revendication 17, choisi parmi:
- un composé selon la revendication 1, dans lequel Ar est un groupe 1- ou 2-naphtalényle;
- un composé selon la revendication 1, dans lequel Y est un atome d'oxygène;
- un composé selon la revendication 1, dans lequel m est un nombre entier de 5 à 7;
- un composé selon la revendication 1, dans lequel m vaut 6;
- le composé selon la revendication 1, qui est la méthyl-5-[6-(2-naphtalényloxy)hexyloxy]-2-furylcétone;
- le composé selon la revendication 1, qui est la méthyl-5-(6-phénoxyhexyloxy)-2-furylcétone;
- le composé selon la revendication 1, qui est la méthyl-5-(10-phénoxydécyloxy)-2-furylcétone.

19. Utilisation d'un composé selon l'une quelconque des revendications 1 à 11, dans laquelle les infections rétrovirales incluent ces maladies provoquées par le virus de la leucémie murine, le virus de la leucémie féline, le virus du sarcome aviaire, le virus immuno-déficitaire humain (HIV), HTLV-I, et HTLV-II.

20. Composition pharmaceutique comprenant le composé de la revendication 1 en mélange avec un support acceptable sur le plan pharmaceutique.

21. Composition pharmaceutique selon la revendication 20, comprenant un composé choisi parmi:
- un composé selon la revendication 1, dans lequel Ar est un groupe 1- ou 2-naphtalényle;
- un composé selon la revendication 1, dans lequel Y est un atome d'oxygène;
- un composé selon la revendication 1, dans lequel m est un nombre entier de 5 à 7;
- un composé selon la revendication 1, dans lequel m vaut 6;
- le composé selon la revendication 1, qui est la méthyl-5-[6-(2-naphtalényloxy)hexyloxy)-2-furylcétone;
- le composé selon la revendication 1, qui est la méthyl-5-(6-phénoxyhexyloxy)-2-furylcétone;
- le composé selon la revendication 1, qui est la méthyl-5-(10-phénoxydécyloxy-2-furylcétone.

## Revendications (Revendications pour l'(les) Etat(s) contractant(s) suivant(s): ES, GR)

1. Procédé pour la préparation d'un composé de formule 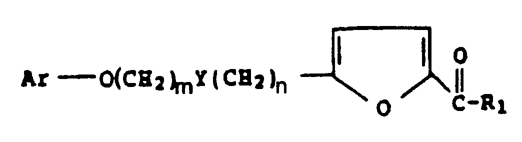 dans laquelle Y représente une liaison, un atome d'oxygène ou un atome de soufre divalent; Ar représente un groupe phényle ou naphtalényle; n vaut 0 ou 1; m est un nombre entier de 4 à 10; et R₁ représente un groupe alkyle en C₁ à C₆; qui comprend les opérations consistant:
a. à traiter un équivalent d'un dérivé d'acide carboxylique de formule 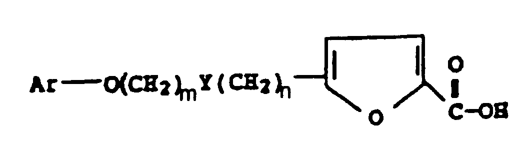 dans laquelle Y, Ar, m et n ont les significations définies ci-dessus, avec deux équivalents d'alkyllithium, dans lesquels le groupe alkyle correspond au substituant R₁ souhaité, dans un solvant choisi parmi l'éther, le tétrahydrofuranne, le p-dioxanne, le diméthoxyéthane et le diéthylèneglycol diméthyléther, à une température comprise entre -10°C et la température de reflux du solvant; ou
b. à combiner un bromure d'alkylmagnésium de formule R₁MgBr et le dérivé imidazolide d'un acide 2-furancarboxylique de formule 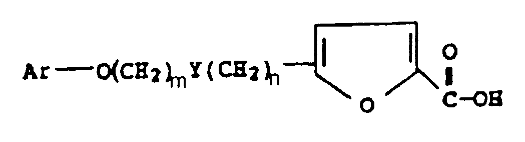 dans laquelle Y, Ar, m et n ont les significations définies ci-dessus, dans un solvant choisi parmi l'éther, le tétrahydrofuranne, le dioxanne, le diméthoxyéthane et l'acétonitrile, et à chauffer le mélange à une température comprise entre 25°C et la température de reflux du solvant; ou
c. à faire réagir un furanne substitué par ArO(CH₂)ₘY(CH₂)ₙ de formule 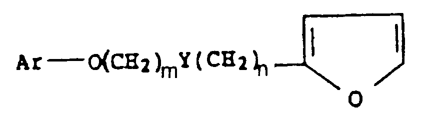 dans laquelle Ar, m, n et Y ont les significations définies ci-dessus, avec un halogénure d'acyle de formule 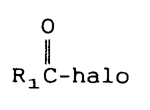 dans laquelle halo est un atome de chlore ou de brome et R₁ a la signification définie ci-dessus, en présence d'un catalyseur acide choisi parmi le trifluorure de bore-éthérate, le chlorure stannique, le chlorure de zinc, l'acide chlorhydrique ou l'acide orthophosphorique.

2. Procédé selon la revendication 1, dans lequel Ar est un groupe 1- ou 2-naphtalényle.

3. Procédé selon la revendication 1, dans lequel Y est l'atome d'oxygène.

4. Procédé selon la revendication 1, dans lequel m est un nombre entier de 5 à 7.

5. Procédé selon la revendication 1, dans lequel m vaut 6.

6. Procédé selon la revendication 1, dans lequel le composé est la méthyl-5-[6-(2-naphtalényloxy)hexyloxy]-2-furylcétone.

7. Procédé selon la revendication 1, dans lequel le composé est la méthyl-5-(6-phénoxyhexyloxy)-2-furylcétone.

8. Procédé selon la revendication 1, dans lequel le composé est la méthyl-5-(10-phénoxydécyloxy)-2-furylcétone.
